(19) 

Europäisches
Patentamt
European
Patent Office
Office européen
des brevets

(11)     **EP 3 868 867 A1**

(12)     **EUROPEAN PATENT APPLICATION**
published in accordance with Art. 153(4) EPC

(43) Date of publication:
**25.08.2021  Bulletin 2021/34**

(21) Application number: **19874694.3**

(22) Date of filing: **16.10.2019**

(51) Int Cl.:
***C12N 1/20*** *(2006.01)*

(86) International application number:
**PCT/JP2019/040702**

(87) International publication number:
**WO 2020/080413 (23.04.2020 Gazette 2020/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.10.2018   JP 2018197010**

(71) Applicant: **Idemitsu Kosan Co., Ltd.**
**Chiyoda-ku**
**Tokyo 100-8321 (JP)**

(72) Inventors:
• INAI, Koji
  **Tsukuba-shi, Ibaraki 300-2646 (JP)**
• EGUCHI, Takanori
  **Tsukuba-shi, Ibaraki 300-2646 (JP)**

(74) Representative: **Vossius & Partner**
**Patentanwälte Rechtsanwälte mbB**
**Siebertstraße 3**
**81675 München (DE)**

(54)     **PLANT RESIDUE-DECOMPOSING AGENT USING LIQUID CULTURE OF BACILLUS PUMILUS KS-C4 STRAIN**

(57)     A plant residue-decomposing agent comprising cells of *Bacillus pumilus* KS-C4 strain (FERM BP-10842), wherein the cells are formulated into the agent by using a liquid culture product of the KS-C4 strain as it is, or by concentrating the liquid culture product of the KS-C4 strain and then without separating the cells from a liquid.

EP 3 868 867 A1

**Description**

TECHNICAL FIELD

The present invention relates to a plant residue-decomposing agent containing *Bacillus pumilus* strain for efficiently decomposing a plant residue left after crop harvest and to a method for decomposing such plant residue using the same.

BACKGROUND ART

Crops cultivated as agricultural products are roughly separated into edible parts and plant residue after harvesting. The plant residue is further separated into those that are collected with the harvested part and those that are left in the field. The collected residue may be provided for industrial use as paddy straw after being dried, utilized as organic fertilizer after undergoing a process such as natural fermentation, or disposed of as waste after undergoing a process such as incineration. On the other hand, the plant residue left in the field is either forced to accelerate natural decomposition by, for example, plowing the plant residue into the soil by some means such as plowing machine, or left untreated. The plant residue, either collected or left in the field, is required to be decomposed in a short period of time from the viewpoint of improving the efficiency of the disposal process and of preparing an efficient working environment for the next planting season. Furthermore, as the plant residue left in the field are returned to the soil through decomposition, they become a nutrient for agricultural products such as crops to be planted in the next season. Therefore, if the plant residue is decomposed in a short period and becomes a nutrient in the soil, an additional application of a chemical fertilizer in an excessive amount can be avoided. Considering these, decomposing the plant residue is important in the aim of accomplishing environmental conservation-oriented agriculture.

Under such circumstances, there has been proposed a method for decomposing and reducing the volume of the plant residue using microorganisms such as *Bacillus* sp. that is capable of decomposing cellulose. For example, Patent document 1 discloses a material for decomposing and reducing volume of the plant residue using *Bacillus pumilus* KS-C4 strain.

RELATED ART DOCUMENT

PATENT DOCUMENT

    [Patent document 1] Japanese Patent No. 4904122

SUMMARY OF THE INVENTION

PROBLEMS TO BE SOLVED BY THE INVENTION

The *Bacillus pumilus* KS-C4 strain disclosed in Patent Document 1 has an outstanding ability to decompose plant residue. Nevertheless, there was still a room for improvement in culturing process and formulation process of the strain so that the decomposition ability of the strain is further enhanced. Accordingly, an object of the present invention is to provide a plant residue-decomposing agent having an enhanced ability to decompose plant residue and applicable to a wide range of plant residues.

MEANS FOR SOLVING THE PROBLEMS

The present inventors have conducted intensive studies to solve the above-mentioned problems, and as a result, obtained the following findings: in formulating a plant residue-decomposing agent using cells of *Bacillus pumilus* KS-C4 strain, by culturing the cells in a liquid culture medium that induces higher cellulase production and using the resultant liquid culture product as it is, or by formulating the cells after concentrating the liquid culture product to such an extent that the cells are not completely separated from the liquid culture medium, the ability to decompose the plant residue can be enhanced, and the plant residue-decomposing agent can be applicable to a broad spectrum of plant residue, thereby completed the invention.

Specifically, the present invention provides the followings:

    [1] A plant residue-decomposing agent containing cells of *Bacillus pumilus* KS-C4 strain (FERM BP-10842), wherein the cells are formulated into the agent by using a liquid culture product of the KS-C4 strain as it is, or by concentrating the liquid culture product of the KS-C4 strain and then without separating the cells from a liquid.

[2] The plant residue-decomposing agent according to [1], wherein the cells are obtained by concentrating the liquid culture product and then drying it.

[3] The plant residue-decomposing agent according to [1], wherein the plant residue-decomposing agent is in a form of granular formulation, liquid formulation, dust formulation, wettable powder, or oil solution.

[4] The plant residue-decomposing agent according to any one of [1] to [3], wherein plant species to be treated comprise vegetables, turfs, weeds, cereals, tubers, legumes, and fruits.

[5] A method for decomposing a plant residue, comprising treating the plant residue with the plant residue-decomposing agent according to any one of [1] to [4].

[6] The method according to [5], wherein the plant residue is directly treated with the plant residue-decomposing agent and/or soil in which the plant residue is remained is treated with the plant residue-decomposing agent.

[7] A method for producing a plant residue-decomposing agent containing cells of *Bacillus pumilus* KS-C4 strain, comprising:

culturing the *Bacillus pumilus* KS-C4 strain in a liquid medium;
concentrating a liquid culture product obtained from the culturing step; and
performing a formulation process by using a concentrated product obtained from the concentrating step without separating the cells from a liquid.

[8] The method according to [7], wherein the formulation process comprises drying the concentrated product.

[9] A dried product of a cultured cell product of *Bacillus pumilus* KS-C4 strain (FERM BP-10842), wherein the cultured cell product is obtained by concentrating a liquid culture product of the KS-C4 strain and then drying the resultant concentrated liquid culture product without separating the cells from a liquid.

EFFECTS OF THE INVENTION

The microbial formulation containing *Bacillus pumilus* KS-C4 strain according to the present invention shows an enhanced ability to decompose plant residue by virtue of its increased heat-resistant cell count and a higher cellulase activity, thereby allowing the plant residue to decompose highly efficiently. The plant residue-decomposing agent of the present invention can make a broad spectrum of residues of plants including not only vegetables, mowed turf grasses, thatch, and weeds, but also cereals such as wheat, maize, and rice, tubers such as potato, legumes such as soybean, and fruit trees readily decomposable. The present invention contributes to the accomplishment of efficient crop cultivation and the promotion of sustainable circular agriculture.

MODES FOR CARRYING OUT THE INVENTION

The plant residue-decomposing agent of the present invention is characterized in that it contains cells of *Bacillus pumilus* KS-C4 strain (FERM BP-10842), and that the cells are formulated into the plant residue-decomposing agent by using a liquid culture product of the KS-C4 strain as it is, or by concentrating the liquid culture product and then without separating the cells from a liquid. As used herein, the phrase "without separating" means that such an operation as a centrifugal separation that separates the cells and a liquid is not performed.

The *Bacillus pumilus* KS-C4 strain is a strain isolated by the present inventors from the soil of a golf course in Saitama Prefecture, Japan, and is characterized by having an ability to decompose plant residue.

The KS-C4 strain has been deposited to the National Institute of Advanced Industrial Science and Technology, Patent Biological Depositary Center (1-1-1-Chuo 6 Higashi, Tsukuba City, Ibaraki Pref., Japan), which is currently National Institute of Technology and Evaluation, International Patent Organism Depositary (IPOD) (2-5-8-120, Kazusakamatari, Kisarazu City, Chiba Pref., Japan) under accession number FERM P-20978 on Aug. 2, 2006, which was then transferred to international deposit under Budapest Treaty and was assigned with accession number FERM BP-10842.

The mycological characteristics of *Bacillus pumilus* KS-C4 strain are as follows:

The KS-C4 strain shows cellulase activity and pectinase activity. As used herein, the phrase "show cellulase activity and pectinase activity" means that the strain produces those enzymes to such an extent that cellulase activity and pectinase activity can be detected in a culture product obtained by culturing the cells of the strain. Specifically, it means that cellulase and pectinase are produced inside the cell and the produced cellulase and pectinase are attached to the outer cell wall or are secreted to the extracellular environment.

Cellulase is an enzyme that hydrolyzes $\beta1\rightarrow4$ glucosidic bond of cellulose. Cellulase include both endoglucanase, which cleaves cellulose chains of the cellulose in a random manner, and cellobiohydrolase, which cleaves cellulose chains of the cellulose from its reducing terminal and produces cellobiose. Pectinase is an enzyme that hydrolyze $\alpha1{\rightarrow}4$ bond of polygalacturonic acid, which acid is a constituent of pectin, pectinic acid, and pectic acid.

By virtue of such enzymatic action, when cells producing these enzymes are introduced into a plant residue, the cells grows by utilizing polysaccharides that constitute the plant in the plant residue as a source of nutrition. As a result, the weight and the volume of the solid content of the plant residue can be reduced.

The DNA base sequence of 16S rRNA gene of the KS-C4 strain is shown in SEQ ID NO: 1.

The KS-C4 strain to be used in the present invention may be a mutant strain derived from the KS-C4 strain having the sequence designated as SEQ ID NO: 1, as long as it shows an ability to decompose plant residue. For example, even if the DNA base sequence of the 16S rRNA gene of the mutant strain differs by one to several bases, such as by one to five bases, by one to three bases, by one to two bases, or by one base as compared to the sequence designated as SEQ ID NO: 1, such mutant strain may fall within the range of the strain referred to as "KS-C4 strain" to be contained in the plant residue-decomposing agent of the present invention, as long as it shows an ability to decompose plant residue equivalent to or greater than that of the original KS-C4 strain with 16S rRNA gene having the base sequence designated as SEQ ID NO: 1.

The mutant strain of the KS-C4 strain may be generated by spontaneous mutation or by inducing mutation by exposing the KS-C4 strain to mutagens such as chemical mutagen or ultraviolet radiation. The KS-C4 strain of the present invention may be obtained among such mutant strains by selecting ones that shows an ability to decompose plant residue equivalent to or enhanced over that of the original KS-C4 strain.

The plant residue-decomposing agent of the present invention may be obtained by culturing the cells of the KS-C4 strain in a liquid culture medium that induces higher cellulase production, followed by formulating the resultant liquid culture product into the decomposing agent either as it is or after concentrating the liquid culture product and then without separating the cells from a liquid.

Typically, the KS-C4 strain may be cultured in a liquid medium by a method used for culturing a *Bacillus pumilus* strain. For example, the culture in the liquid medium is typically carried out at a temperature of 10 to 50°C, preferably 30 to 40°C. The duration of the culture is not particularly limited as long as cells of interest can be obtained in a sufficient amount. By way of example, the culture is carried out for 20 to 72 hours. The method for culture may be a method for liquid culture such as reciprocal shaking culture and jar fermenter culture.

As the liquid medium for the culture, a common liquid culture medium containing a medium component such as a carbon source and a nitrogen source in an appropriate concentration may be used.

Examples of the carbon source include saccharides (such as starch, glucose, lactose, glycerol, arabinose, ribose, xylose, galactose, fructose, mannose, inositol, mannitol, sorbitol, glucosamine, N-acetylglucosamine, cellobiose, maltose, sucrose, trehalose, xylitol) or sugar sources, alcohols, organic acids, organic acid salts, alkanes, or other common carbon sources. Examples of the nitrogen source include soy derived ingredients, yeast derived ingredients, maize derived ingredients, animal and plant proteins and the decomposition products thereof, ammonium salts such as ammonium nitrate, ammonium sulfate, ammonium chloride, and ammonium acetate, ammonia, sodium nitrate, potassium nitrate, sodium glutamate, urea, and the like. Examples of the medium component other than carbon source and nitrogen source include trace metal salts, amino acids, vitamins, and the like. Where appropriate, an anti-foaming agent may be added if required for efficient culturing. Among such liquid media, a medium containing both of a nitrogen source and a carbon source at high concentrations, i.e., a carbon source selected from those mentioned above at 1% by weight or more as well as a nitrogen source selected from those mentioned above at 1% by weight or more is desirable.

After the culture is completed, the resultant liquid culture product containing the liquid culture medium and the cells may be used for formulation as it is. However, for the purpose of increasing the concentration of the cells before the formulating process, the resultant liquid culture product is preferably subjected to a concentrating process to such an extent that the cells and the liquid medium are not separated each other. As used herein the term "concentrating" refers to any operation that reduces the volume of the liquid medium while keeping the cells contained therein and maintaining the total amount of the enzyme secreted into the liquid medium, particularly cellulase, at a certain level. The concentrating process may be accomplished by such techniques as centrifuge, filtration, a method using ultrafiltration, and concentrating under reduced pressure using an evaporator. The concentrating process may also be accomplished by a drying process.

In order to concentrate the liquid culture product with maintaining the amount of the cells at a certain level while at the same time keeping the activity of the secreted enzyme, especially cellulase, at a high level, the total volume of the liquid medium is preferably reduced to a half or less, more preferably to a quarter or less, and further preferably to one-eighth or less of the initial volume used for culturing.

This concentrating process is performed in such a manner that the cells are not completely separated from the liquid medium so that the cells are formulated along with the liquid medium. In the concentrated product obtained from the concentrating process, the liquid medium is preferably remained in an amount of equal to or more than one-four hundredth of the initial volume used for culturing. If a particularly high cellulase content is desired, the liquid medium is preferably remained in an amount of equal to or more than hundredth of the initial volume.

When the plant residue-decomposing agent of the present invention is prepared as a liquid formulation, the liquid culture product may be used for formulation as it is, or the concentrated product obtained from the concentrating process containing the cells may be used for the formulation as it is. Typically, the liquid culture product or the concentrated product thereof is preferably dried prior to the formulating process. This drying process is preferably carried out such that the water content of the plant residue-decomposing agent is 10% by mass or lower. The method for drying is not particularly limited, and the examples of the drying method include natural drying, forced-air drying, spray drying, and freeze drying. Among these, spray drying or freeze drying is preferably used. During the drying, a protective agent such as skim milk, sodium glutamate, and saccharides may be used.

When the concentrated product is dried prior to the formulating process, the enzyme concentration of cellulase in the dried, concentrated product is preferably 10 to 10000 U/g, and more preferably 100 to 10000 U/g. In addition, the concentration of the cells in the dried, concentrated product is preferably $1 \times 10^6$ to $1 \times 10^{13}$ CFU/g, and more preferably $1 \times 10^7$ to $5 \times 10^{12}$ CFU/g.

The unit used to express the cellulase activity, i.e., one unit (U), is defined as the amount of enzyme capable of converting a substrate of cellulase (such as carboxymethyl cellulose or a cellulose filter paper) to produce a reducing sugar equivalent to 1 $\mu$mol of glucose per minute under a specified condition at a pH of between 6.0 to 7.0 at a temperature of 25°C to 30°C. The measurement of the cellulase activity may be performed by determining the amount of the reducing sugar using high performance liquid chromatography (HPLC), or by determining the amount of the reducing sugar by a colorimetric technique such as Somogyi-Nelson method or DNS method. Alternatively, an assay kit such as MarkerGene (trademark) Fluorescent Cellulase Assay Kit using a fluorescent substrate may be used, whereby the cellulase activity may be determined based on a standard curve prepared using a purified enzyme.

The liquid culture product, the concentrated product thereof, or the dried product thereof as described above may be formulated as a plant residue-decomposing agent after optional dilution.

The cell concentration of the KS-C4 strain per unit weight of the plant residue-decomposing agent according to the present invention is preferably $1 \times 10^3$ to $1 \times 10^{13}$ CFU/g, and more preferably $1 \times 10^4$ to $5 \times 10^{12}$ CFU/g. For example, in an embodiment in which the plant residue-decomposing agent is diluted prior to application to plant residue, the cell concentration is preferably $1 \times 10^7$ to $5 \times 10^{12}$ CFU/g. In an embodiment in which the plant residue-decomposing agent is applied to plant residue without being diluted, the cell concentration is preferably $1 \times 10^4$ to $1 \times 10^7$ CFU/g.

As regards to the enzyme concentration per unit weight of the plant residue-decomposing agent according to the present invention, the cellulase concentration is preferably 0.001 to 10000 U/g, and more preferably 0.001 to 5000U/g. For example, in an embodiment in which the plant residue-decomposing agent is diluted prior to application to plant residue, the cellulase concentration is preferably 1 to 10000 U/g. In an embodiment in which the plant residue-decomposing agent is applied to plant residue without being diluted, the cellulase concentration is preferably 0.001 to 10 U/g.

The cells of the KS-C4 strain contained in the plant residue-decomposing agent of the present invention may be in the state of a spore or in the state of a vegetative cell. Typically, from the viewpoint of storage stability and thermal stability, it is preferable that the cells be in the state of a spore. For inducing spore-forming, culture conditions such as the composition of the medium, pH of the medium, and the temperature, the humidity, and the oxygen content at which the culture is carried out may be tailored to the spore-forming conditions during the cell culture cycle.

The liquid culture product, or the concentrated product thereof obtained by the concentrating process containing the cells and the concentrated liquid component, or the dried product thereof may be formulated as it is, or may be admixed with an additional material such as a carrier prior to be formulated.

For example, any material such as a liquid carrier, a solid carrier, a surfactant (emulsifier, dispersant, anti-foaming agent, and the like), or an auxiliary agent may be added to the above-described liquid culture product, the concentrated product thereof, or the dried product thereof prior to the formulating process. In other words, the plant residue-decomposing agent of the present invention may include an additional material such as a liquid carrier, a solid carrier, a surfactant (emulsifier, dispersant, anti-foaming agent, and the like), or an auxiliary agent.

Such additional material is not particularly limited as long as it is environmentally safe. Any material commonly used in a formulation for soil application or a fertilizer may be used. Examples of the liquid carrier include a phosphate buffer, a carbonate buffer, and physiological saline, and the like. Examples of the solid carrier include powders of natural minerals such as kaolin, clay, talc, chalk, quartz, palygorskite (attapulgite), montmorillonite, and diatomaceous earth; powders of synthetic minerals such as silicic acid, alumina, and silicate; and a natural polymeric products such as crystalline cellulose, corn starch, gelatin, and alginic acid. Examples of the surfactant include an ester of polyoxyethylene and a fatty acid, an ester of polyoxyethylene and a fatty alcohol, alkyl aryl polyglycol ether, alkyl sulfonate, alkyl sulfate, aryl sulfonate, and the like. Examples of the auxiliary agent include glycerol, carboxymethyl cellulose, polyoxyethyleneglycol, acacia gum, starch, lactose, and the like.

The dosage form of the plant residue-decomposing agent of the present invention is not particularly limited as long as it is suitable for a conventional microbial formulation. Examples of such dosage form include granular formulation, liquid formulation, dust formulation, wettable powder, or oil solution.

The plant residue-decomposing agent of the present invention can decompose a plant residue by treating the plant residue therewith. As used herein "plant residue" refers to a material that contains at least a part of a plant such as a leaf, a stem, a flower, and a root, and needs to be get rid of and to be decomposed. Examples of such plant residue include mowed turf grass and thatch; wood material such as sawdust, bark, pruned branch; straw such as rice straw and wheat straw; crop residue such as chaff and bran; harvest residue of agricultural crop and the like; root residue of agricultural crop and the like remained in the soil; domestic food waste containing a plant such as a vegetable; feces of herbivorous livestock; and food industrial waste.

Among these plant residues, the plant residue-decomposing agent of the present invention is particularly suited for decomposing a harvest residue of agricultural crop and the like, in particular for a residue of cereals such as wheat, maize, and rice, tubers such as potato, and legumes such as soybean.

The treatment with the plant residue-decomposing agent of the present invention may be carried out by directly treating the plant residue of interest or by treating the soil where the plant residue remains therein. The plant residue-decomposing agent may be applied in an appropriate manner which varies depending on the factors including the location and the size of the place where the plant residue of interest exists and the dosage form of the decomposing agent. For example, the plant residue-decomposing agent may be applied to a pile of plant residues, or may be sprayed onto or admixed with the soil where the plant residue exists. The plant residue-decomposing agent may be diluted prior to application, if necessary.

The amount of the plant residue-decomposing agent of the present invention to be used is not particularly limited as long as the KS-C4 strain keeps the number of cells that enables the cells to survive and proliferate. For example, it is preferable to apply the plant residue-decomposing agent in a concentration typically of 0.1 to 50 $g/m^2$, and preferably 0.5 to 10 $g/m^2$, or 0.1 to 100 $g/m^3$, and preferably 0.5 to 20 $g/m^3$, as expressed on dry powder basis.

EXAMPLES

The present invention will be described more specifically hereinbelow by way of Examples. However, the following Example is only for illustrating an aspect of the present invention, which is not limited to the embodiment described hereinbelow.

[Preparation of dried bacterial cell]

Colony of the KS-C4 strain was grown on nutrient agar medium, and one platinum loop was inoculated into 100 mL of the medium described in Table 1. Each medium was prepared in a volume of 100 mL and sterilized by autoclave beforehand. In order to avoid Maillard reaction, glucose was separately sterilized, and then aseptically admixed with the sterilized medium. After the inoculation, the inoculated medium was cultured with shaking at 150 rpm at a temperature of 30°C for 44 hours. Subsequently, the resultant liquid culture product was collected to a volume of 2 L and was centrifuged at 3000 rpm for 15 minutes, thereby yielding 500 mL of a concentrated cell solution (i.e., 1500 mL of supernatant was discarded). The thus obtained concentrated solution containing the cells was dried with a spray drier, to thereby obtain the dried cells.

Table 1

| Medium composition | | |
|---|---|---|
| ingredient | supplier | concentration (g/L) |
| glucose | Wako Junyaku | 25.0 |
| defatted soybean flour | Ajinomoto Healthy Supply Co., Inc. | 20.0 |
| corn steep liquor | Difco | 5.0 |
| yeast extract | Roquette | 4.0 |
| $MnCl_2 \cdot 4H_2O$ | Difco | 0.18 |
| NaCl | Wako Junyaku | 1.00 |
| $KH_2PO_4$ | Wako Junyaku | 0.50 |
| $MgSO_4 \cdot 7H_2O$ | Wako Junyaku | 0.63 |
| $CaCl_2$ | Wako Junyaku | 0.19 |
| $FeSO_4$ | Wako Junyaku | 0.00038 |

(continued)

| Medium composition | | |
|---|---|---|
| ingredient | supplier | concentration (g/L) |
| KM-70 | Shin-Etsu Chemical Co., Ltd. | 1.25 |

[Measurement of heat-resistant cell count]

In the course of the above-described preparation process, the heat-resistant cell count was measured on the liquid culture, the concentrated product of the liquid culture, and the dried product of the concentrated product, respectively, in the method described hereinbelow. The results are shown in Table 2. The heat-resistant cell count could be increased by 100 times or more by concentrating and drying the cell culture product.
The method for measuring the heat-resistant cell count is as follows:

Each sample was incubated at a temperature of 80°C for 10 minutes. Subsequently, heat-resistant cell count was determined by dilution plate method. For measuring a spray-dried sample, the sample was diluted with sterilized water before the measurement.

Table 2: Heat-resistant cell count per 1 g of sample (based on the cell count of the liquid culture, expressed as 100%)

| | liquid culture | after centrifuging and discarding the supernatant (concentrated product) | after spray-drying (dried product) |
|---|---|---|---|
| heat-resistant cell count (cfu/g) | 100% | 170% | 10560% |

[Measurement of cellulase activity]

As a comparative example, the KS-C4 strain was cultured in broth medium (liquid medium supplemented with meat extract, peptone, $KH_2PO_4$, and $MgSO_4$) at a temperature of 30°C for 2 days. The resultant liquid culture product was used as an inoculum, and was inoculated into whole soybean seed as a substrate for solid culture (sterilized at a temperature of 121°C for 30 minutes by autoclave beforehand), followed by culturing at a temperature of 30°C for 2 days. The thus-obtained each solid culture of the strain was air-dried at room temperature, and was pulverized with a mill, to thereby obtain a dried cell.
Cellulase activity of the dried cells obtained in Example above by concentrating and drying the liquid culture product of the KS-C4 strain was measured, and the measured cellulase activity was compared to that of the dried cells obtained from the comparative example.
Specifically, each of the dried cells in equal weight was diluted, and then spread onto agar medium containing CMC (carboxymethylcellulose). After culturing for a certain period of time, the medium was stained with congo red and decomposition of CMC was detected by the formation of clear halo.
The results are shown in Table 3. The results show that the cellulase activity of the concentrated and dried product of the liquid culture product was considerably higher.

Table 3: Cellulase activity of solid culture and liquid culture

| | dilution ratio | activity (size of halo zone) |
|---|---|---|
| dried product of solid culture | ×10 | 6 |
| | ×20 | 0 |
| | ×50 | 0 |
| | ×100 | 0 |
| dried product of liquid culture | ×10 | 10.31 |
| | ×20 | 9.91 |
| | ×50 | 9.23 |
| | ×100 | 8.54 |

[Plant residue decomposition test]

The plant residues used for this test are as follows:

| rice | variety: Koshihikari | cultivated in a pot containing nursery soil for paddy rice seedlings (Heisei Baido), and the above-ground parts were collected and dried |
| wheat | variety: Satonosora | cultivated in a pot containing horticultural soil (Genki-kun No.1), and the above-ground parts were collected and dried |
| soybean | variety: Yukihomare | cultivated in a pot containing horticultural soil (Genki-kun No.1), and the above-ground parts were collected and dried |
| potato | variety: Toyoshiro | cultivated in a pot containing horticultural soil (Genki-kun No.1), and the leaves were collected and dried |
| maize | variety: 38V52 | cultivated in a pot containing horticultural soil (Genki-kun No.1), and the above-ground parts were collected and dried |

The dried plant residues were cut into pieces of 1 to 2 cm in size, which were then immersed in sterilized water. Subsequently, each formulation described below was added thereto at a cell concentration of $10^8$ cfu per 1 g (on dry basis) of the plant residue, followed by culturing at 30°C for 30 days.

After the culturing, the resultant plant residue was collected and dried at 80°C, and the dry weight was measured.

This test was carried out in triplicate for each plot, and the dry weight of the plant residue obtained after the treatment was averaged over the triplicate measurements for each plot. The reduction rate of the averaged weight of the plant residue with respect to the averaged weight before the treatment was calculated, which is defined as "decomposition rate."

$$decomposition\ rate\ (\%) = (average\ dried\ weight\ of\ plant\ residue\ after$$

$$treatment/average\ dried\ weight\ of\ plant\ residue\ before\ treatment) \times 100$$

The results are shown in Tables 4 to 8. The dust formulation 1 was used as the dust formulation in the Tables.

The decomposing agent of the present invention has shown significantly higher decomposition rate on all types of plant residues as compared to the decomposing agent prepared from the solid culture.

Table 4: Decomposition activity test on rice plant residue (decomposition rate)

| | dosage form | | | |
|---|---|---|---|---|
| active ingredient | wettable powder | oil solution | dust formulation | liquid formulation |
| liquid culture | 75% | 67% | 48% | 49% |
| solid culture | 36% | 48% | 31% | 43% |

Table 5: Decomposition activity test on wheat plant residue (decomposition rate)

| | dosage form | | | |
|---|---|---|---|---|
| active ingredient | wettable powder | oil solution | dust formulation | liquid formulation |
| liquid culture | 69% | 55% | 72% | 53% |
| solid culture | 49% | 37% | 20% | 30% |

Table 6: Decomposition activity test on soybean plant residue (decomposition rate)

| | dosage form | | | |
|---|---|---|---|---|
| active ingredient | wettable powder | oil solution | dust formulation | liquid formulation |
| liquid culture | 67% | 75% | 58% | 60% |
| solid culture | 47% | 48% | 35% | 43% |

Table 7: Decomposition activity test on potato plant residue (decomposition rate)

| | dosage form |
|---|---|
| active ingredient | wettable powder |
| liquid culture | 73% |
| solid culture | 50% |

Table 8: Decomposition activity test on maize plant residue (decomposition rate)

| | dosage form | | | |
|---|---|---|---|---|
| active ingredient | wettable powder | oil solution | dust formulation | liquid formulation |
| liquid culture | 70% | 70% | 68% | 72% |
| solid culture | 55% | 34% | 37% | 47% |

[Preparation example of formulation]

Colony of the KS-C4 strain was grown on nutrient agar medium, and one platinum loop was inoculated into 100 mL of the medium described in Table 1. Each medium was prepared in a volume of 100 mL and sterilized by autoclave beforehand. In order to avoid Maillard reaction, glucose was separately sterilized, and then aseptically admixed with the sterilized medium. After the inoculation, the inoculated medium was cultured with shaking at 150 rpm at a temperature of 30°C for 44 hours. Subsequently, the resultant liquid culture was collected to a volume of 2 L and was centrifuged at 3000 rpm for 15 minutes, thereby yielding 500 mL of a concentrated bacterial cell solution (i.e., 1500 mL of supernatant was discarded). The thus obtained concentrated solution containing the cells was dried with a spray drier, to thereby obtain the dried bacterial cell.

[Powder formulation 1]

The dried cells were admixed with HA Kaolin clay such that the liquid cultured cells of the KS-C4 strain were contained in the final formulation at a concentration of $5 \times 10^8$ cfu/g, and the resultant mixture was pulverized with a mixer, to thereby obtain the dust formulation.

[Wettable powder]

The dried cells were admixed with HA Kaolin clay, Sorpol 5082, and soybean flour such that the liquid cultured cells of the KS-C4 strain were contained in the final formulation at a concentration of $5 \times 10^8$ cfu/g and Sorpol 5082 and soybean flour were contained in the final formulation at concentrations of 10% and 3%, respectively, to thereby obtain the wettable powder.

[Dust formulation 2]

The dried cells were admixed with soybean flour such that the liquid cultured cells of the KS-C4 strain were contained in the final formulation at a concentration of $5 \times 10^8$ cfu/g and the soybean flour was contained in the final formulation at a concentration of 3%. The resultant mixture was temporarily diluted with an appropriate amount of water, and the resultant mixture was sprayed onto pumice, followed by air-drying, to thereby obtain the dust formulation.

[Emulsifier]

To the dried cells was added emulsifier at a concentration of 5%, and the resultant mixture was admixed with soybean oil such that the liquid cultured cells of the KS-C4 strain were contained in the final formulation at a concentration of $5 \times 10^8$ cfu/g, to thereby obtain the oil solution.

[Liquid formulation]

The dried cells were admixed with glycerol, Tween-20, and water such that the liquid cultured cells of KS-C4 strain were contained in the final formulation at a concentration of $5 \times 10^8$ cfu/g and the glycerol and Tween-20 were contained in the final formulation at a concentration of 10% and 10%, respectively, to thereby obtain the liquid formulation.

[Solid culture]

As a comparative example, the KS-C4 strain was cultured in broth medium (liquid medium supplemented with meat extract, peptone, $KH_2PO_4$, and $MgSO_4$) at a temperature of 30°C for 2 days. The resultant liquid culture product was used as an inoculum, and was inoculated into whole soybean seed as a substrate for solid culture (sterilized at a temperature of 121°C for 30 minutes by autoclave beforehand), followed by culturing at a temperature of 30°C for 2 days. The thus-obtained culture was air-dried at room temperature, and was pulverized with a mill, to thereby obtain a dried bacterial cell. This dried cells were used as a solid culture.

**Claims**

**1.** A plant residue-decomposing agent comprising cells of *Bacillus pumilus* KS-C4 strain (FERM BP-10842), wherein the cells are formulated into the agent by using a liquid culture product of the KS-C4 strain as it is, or by concentrating the liquid culture product of the KS-C4 strain and then without separating the cells from a liquid.

2. The plant residue-decomposing agent according to claim 1, wherein the cells are obtained by concentrating the liquid culture product and then drying it.

3. The plant residue-decomposing agent according to claim 1, wherein the plant residue-decomposing agent is in a form of granular formulation, liquid formulation, dust formulation, wettable powder, or oil solution.

4. The plant residue-decomposing agent according to any one of claims 1 to 3, wherein plant species to be treated comprise vegetables, turfs, weeds, cereals, tubers, legumes, and fruits.

5. A method for decomposing a plant residue, comprising treating a plant residue with the plant residue-decomposing agent according to any one of claims 1 to 4.

6. The method according to claim 5, wherein the plant residue is directly treated with said plant residue-decomposing agent and/or soil in which the plant residue is remained is treated with said plant residue-decomposing agent.

7. A method for producing a plant residue-decomposing agent comprising cells of *Bacillus pumilus* KS-C4 strain, comprising:

   culturing the *Bacillus pumilus* KS-C4 strain in a liquid medium;
   concentrating a liquid culture product obtained from the culturing step; and
   performing a formulation process by using a concentrated product obtained from the concentrating step without separating the cells from a liquid.

8. The method according to claim 7, wherein the formulation process comprises drying the concentrated product.

9. A dried product of a cultured cell product of *Bacillus pumilus* KS-C4 strain (FERM BP-10842), wherein the cultured cell product is obtained by concentrating a liquid culture product of the KS-C4 strain and then drying the resultant concentrated liquid culture product without separating the cells from a liquid.

## INTERNATIONAL SEARCH REPORT

| International application No. |
| --- |
| PCT/JP2019/040702 |

**A. CLASSIFICATION OF SUBJECT MATTER**
Int.Cl. C12N1/20(2006.01)i

According to International Patent Classification (IPC) or to both national classification and IPC

**B. FIELDS SEARCHED**

Minimum documentation searched (classification system followed by classification symbols)
Int.Cl. C12N1/20

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN), GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
| --- | --- | --- |
| X | WO 2008/041505 A1 (IDEMITSU KOSAN CO., LTD.) 10 April 2008, abstract, paragraphs [0008], [0013], [0015], [0016], [0018], [0020]–[0022] & JP 2008-86287 A & US 2010/0028974 A1, abstract, paragraphs [0013], [0022], [0028], [0029], [0032], [0033], [0035]–[0039] & EP 2062969 A1 | 1–9 |

☒ Further documents are listed in the continuation of Box C.    ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 10 December 2019 (10.12.2019) | 24 December 2019 (24.12.2019) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2019/040702

C (Continuation).   DOCUMENTS CONSIDERED TO BE RELEVANT

| Category* | Citation of document, with indication, where appropriate, of the relevant passages | Relevant to claim No. |
|---|---|---|
| A | JP 4-079882 A (SHOWA DENKO KABUSHIKI KAISHA) 13 March 1992, page 3, lower right column, line 5 to page 4, upper left column, line 10 & US 5314637 A & US 5318905 A & US 5231022 A & EP 468464 A2, page 6, lines 19-38 | 1-9 |
| A | KOTCHONI, S. O. and SHONUKAN, O. O., "Regulatory mutaions affecting the synthesis of cellulase in Bacillus pumilus", World Journal of Microbiology and Biotechnology, 2002, vol. 18, pp. 487-491, in particular, table 3 | 1-9 |
| A | THOMAS, S. and RUSSELL, A. D., "Studies on the mechanism of the sporicidal action of glutaraldehyde", Journal of Applied Microbiology, 1974, vol. 37, pp. 83-92, in particular, page 84, lines 6-13 | 1-9 |
| A | JP 2015-521029 A (CJ CHEILJEDANG CORPORATION) 27 July 2015, in particular, example 4 & WO 2013/151361 A1 | 1-9 |
| A | HACHISUKA, Y., 芽胞（細菌胞子）の耐熱性の機構, 化学と生物, 1980, vol. 18, no. 11, pp. 731-739, in particular, page 731, non-official translation ("Mechanism of heat resistance of spores (bacterial spores)", KAGAKU TO SEIBUTSU) | 1-9 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4904122 B

## SEQUENCE LISTING

<110>  Idemitsu Kosan Co., Ltd.

<120>  Plant residue decomposing agent using a liquid culture product of
       Bacillus pumilus KS-C4 strain

<130>  1800262-477

<150>  JP2018-197010
<151>  2018-10-18

<160>  1

<170>  PatentIn version 3.5

<210>  1
<211>  532
<212>  DNA
<213>  Bacillus pumilus

<400>  1
gagtttgatc ctggctcagg acgaacgctg gcggcgtgcc taatacatgc aagtcgagcg      60
aacagaaggg agcttgctcc cggatgttag cggcggacgg gtgagtaaca cgtgggtaac     120
ctgcctgtaa gactgggata actccgggaa accggagcta ataccggata gttccttgaa     180
ccgcatggtt caaggatgaa agacggtttc ggctgtcact tacagatgga cccgcggcgc     240
attagctagt tggtggggta atggctcacc aaggcgacga tgcgtagccg acctgagagg     300
gtgatcggcc acactgggac tgagacacgg cccagactcc tacgggaggc agcagtaggg     360
aatcttccgc aatggacgaa agtctgacgg agcaacgccg cgtgagtgat gaaggttttc     420
ggatcgtaaa gctctgttgt tagggaagaa caagtgcgag agtaactgct cgcaccttga     480
cggtacctaa ccagaaagcc acggctaact acgtgccagc agccgcggta at             532